Europäisches Patentamt

European Patent Office

Office européen des brevets

(19)

(11) **EP 0 834 739 A2**

(12) **EUROPÄISCHE PATENTANMELDUNG**

(43) Veröffentlichungstag:
08.04.1998 Patentblatt 1998/15

(51) Int. Cl.⁶: **G01N 33/18**, G01N 27/49,
G01N 27/48

(21) Anmeldenummer: 97250258.7

(22) Anmeldetag: 04.09.1997

(84) Benannte Vertragsstaaten:
AT BE CH DE DK ES FI FR GB GR IE IT LI LU MC
NL PT SE

(30) Priorität: 04.09.1996 DE 19637704

(71) Anmelder:
LAR Analytik und Umweltmesstechnik GmbH
D-10963 Berlin (DE)

(72) Erfinder: **Pilz, Ulrich, Dr.-Ing.**
13465 Berlin (DE)

(74) Vertreter:
**Christiansen, Henning, Dipl.-Ing.**
**Patentanwalt**
**Pacelliallee 43/45**
**14195 Berlin (DE)**

(54) **Verfahren und Vorrichtung zur Bestimmung der Oxidierbarkeit von Abwasserinhaltsstoffen**

(57) Verfahren zur Bestimmung des chemischen Sauerstoffbedarfs von Wasser bzw. Abwasser über die elektrochemische Oxidation von deren Inhaltsstoffen an einer Bleidioxid-Arbeitselektrode, wobei - die Bleidioxid-Arbeitselektrode zeitlich aufeinanderfolgend bei mindestens zwei verschiedenen vorgegebenen Potentialen betrieben wird, - der bei jedem vorgegebenen Potential durch das Wasser bzw. Abwasser fließende Strom gemessen wird und - die Strom-Meßwerte in eine vorgegebene Beziehung zueinander gesetzt werden, insbesondere ihr Quotient oder ihre Differenz oder eine Meßkurve I = f(U) gebildet wird, wobei das Ergebnis des In-Beziehung-Setzens eine Aussage über das Vorkommen von Wasserinhaltsstoffen mit unterschiedlichem spezifischem Sauerstoffbedarf darstellt sowie eine entsprechende Vorrichtung.

Fig.1

## Beschreibung

Die Erfindung betrifft ein Verfahren der im Oberbegriff des Anspruchs 1 angegebenen Art sowie eine Vorrichtung zur Durchführung des Verfahrens.

Die Bestimmung des chemischen Sauerstoffbedarfs ("CSB"; auch als "COD" = chemical oxygen demand bekannt) spielt bei der Untersuchung von Abwässern zur Bewertung der Oxidierbarkeit der Abwasserinhaltsstoffe - insbesondere mit dem Ziel einer entsprechenden Steuerung der Abwasserbehandlung in einer Kläranlage eine große Rolle. Der Wahl dieses Parameters als herausragende Kenngröße von Abwässern liegt die Überlegung zugrunde, die Oxidierbarkeit sämtlicher Wasserinhaltsstoffe, deren Umsetzung im biologischen Gewässerreinigungsprozeß mit einem Verbrauch von Sauerstoff einhergeht, auf chemischem Wege zu erfassen. Die Bedeutung dieser Größe kommt auch dadurch zum Ausdruck, daß der CSB in nahezu alle im Bereich der Abwasserkontrolle sowie der Gewässer- und Einleiterüberwachung gültige gesetzliche Bestimmungen (beispielsweise das Abwasserabgabengesetz) Eingang gefunden hat.

Bei früher in der Praxis dominierenden Methoden zur Bestimmung des CSB wurden die oxidierbaren Wasserinhaltsstoffe bei aggressiven chemischen Bedingungen (hohe Temperatur, stark saures Milieu) mit einem starken Oxidationsmittel (beispielsweise $K_2MnO_4$ oder $K_2CR_2O_7$) in Kontakt gebracht. Zu diesen Methoden existieren Modifikationen - wozu der Einsatz selektiver Katalysatoren zählt - , welche zum Ziel haben, die Messung bestimmten vorherrschenden Wasserinhaltsstoffen anzupassen.

Diese Verfahren haben jedoch die wesentlichen Nachteile, daß sie arbeitsaufwendig sind und nicht kontinuierlich arbeiten - weshalb sie im Rahmen einer kontinuierlichen Gewässer- oder Einleiterüberwachung wegen des großen Kostenaufwandes einer Untersuchung zahlreicher Einzelproben praktisch nicht einsetzbar sind - und den Einsatz gefährlicher und potentiell stark umweltbelastender Chemikalien erfordern, weshalb die Analysenrückstände aufwendig entsorgt werden müssen. Letzterer Umstand verringert die praktische Bedeutung der traditionellen Verfahren nochmals entscheidend.

In der deutschen Offenlegungsschrift DE 2 135 949 A1 und der US-Patentschrift 3 725 236 beschriebenen Meßverfahren nutzen die Methode der Wasserelektrolyse, wobei die Wasserstoff entwickelnde Elektrode sich in einem getrennten Raum befindet, der über eine Membran elektrisch leitend mit der Wasserprobe verbunden ist, in die die Sauerstoff entwickelnde Elektrode eintaucht. Die Messung beruht darauf, daß die durch einen vorbestimmten Stromfluß theoretisch erzeugbare Sauerstoffmenge mit der tatsächlich erzeugten verglichen wird. Erstere wird über die verbrauchte Ladungsmenge bestimmt, während letztere manometrisch als Druckerhöhung gemessen wird. Bei dieser Methode ist das Meßergebnis systemspezifisch mit erheblichen Fehlern behaftet. Auch ist eine kontinuierliche Messung nicht ohne weiteres möglich.

Ein weiteres in der deutschen Offenlegungsschrift DE 2 137 073 A1 beschriebenes Meßverfahren läuft in einer rohrartigen Meßzelle ab, durch die die zu untersuchende Probe kontinuierlich gefördert wird, nachdem sie zuvor auf 40°C erwärmt und mit einer Mineralsäure angesäuert wurde. Über zwei an der Rohrwandung angebrachte Metallelektroden wird in der Wasserprobe eine Elektrolyse ausgeführt, d.h. an ihnen entwickelt sich theoretisch Sauerstoff bzw. Wasserstoff. Eine dritte Elektrode durchläuft einen vorgegebenen Potentialbereich, der zwischen den elektrochemischen Potentialen der beiden anderen Elektroden liegt, und der durch diese fließende Strom wird als Funktion des Elektrodenpotentials aufgezeichnet. Die zuletzt genannte Elektrode nimmt dabei elektrochemische Bedingungen an, die sowohl reduzierend als auch oxidierend wirken; so ist es je nach Potential möglich, sowohl gelösten Sauerstoff zu reduzieren als auch Wasserinhaltsstoffe direkt zu oxidieren. Dieses Verfahren ist umständlich und ungenau, und in praxi ist ebenfalls eine kontinuierliche Messung kaum möglich.

Aus der europäischen Patentschrift EP 0 282 441 B1 ist ein weiteres Verfahren zur Bestimmung des CSB bekannt. Dieser wird hierbei durch unspezifische elektrochemische Oxidation der in einer Wasserprobe gelösten Stoffe bei kontrolliertem Potential bestimmt, wobei der über eine Bleidioxd-Arbeitselektrode durch die wäßrige Lösung fließende Strom gemessen wird und ein Maß für die je Zeiteinheit an den Elektroden umgesetzte Stoffmenge bildet. Die Methode beruht darauf, daß an einer Bleidioxidelektrode bevorzugt eine Bildung von Ozon und OH-Radikalen erfolgt - die sehr reaktive Oxidationsmittel sind - , und daß deren Bildungsrate und damit auch ihr durch die Oxidation der Inhaltsstoffe einer Probenlösung verursachter Verbrauch sehr einfach und genau durch die Messung des elektrischen Stromflusses durch die Bleidioxidelektrode erfaßt werden kann. Mit diesem Verfahren ist eine praxisgerechte kontinuierliche Messung des CSB mit niedrigem Aufwand möglich.

Im Rahmen von Untersuchungen der Anmelderin hat sich jedoch herausgestellt, daß allein mit dem einzelnen Wert des mit diesem Verfahren bestimmten CSB, der zudem in gewissem Maße von der Wahl des konstantgehaltenen Potentialwertes abhängt, erhöhten Anforderungen hinsichtlich einer möglichst spezifischen Charakterisierung der Wasserinhaltsstoffe nicht hinreichend genügt werden kann.

Der Erfindung liegt daher die Aufgabe zugrunde, ein Verfahren der eingangs genannten Gattung, das spezifischere Aussagen über verschiedene Wasserinhaltsstoffe - speziell im Hinblick auf deren Oxidierbarkeit ermöglicht, sowie eine Vorrichtung zur Durchführung des Verfahrens anzugeben.

Diese Aufgabe wird - was das Verfahren angeht - mit den im Anspruch 1 angegebenen Maßnahmen sowie - was die Vorrichtung angeht - mit den im Anspruch 10 genannten Mitteln gelöst.

Die Erfindung schließt den Gedanken ein, dem gattungsgemäßen Verfahren durch eine die relevanten elektrischen Größen abtastende Ausführung qualitative Aussagemöglichkeiten abzugewinnen.

Sie schließt des weiteren den Gedanken ein, hierzu eine Variation des (bislang auf einen konstanten Wert festgelegten) Potentials unter Messung der zugehörigen Ströme oder alternativ eine - insbesondere ebenfalls variable - Festlegung der Stromstärke und Messung der sich dabei einstellenden Potentiale vorzunehmen. Bereits eine Messung bei zwei Werten der jeweils festgelegten Größe ermöglicht gewisse qualitative Aussagen zum Vorhandensein leicht oder schwer oxidierbareer Stoffe in der Probe, die sich bei Aufnahme regelrechter Meßkurven weiter verfeinern lassen.

Grundsätzlich ermöglicht das erfindungsgemäße Verfahren somit Aussagen über den ungefähren Gehalt an bestimmten Wasser- bzw. Abwasserinhaltsstoffen mit unterschiedlicher Oxidierbarkeit und auf dieser Grundlage eine wesentlich verfeinerte Steuerung von Wasser- bzw. Abwasserbehandlungsanlagen.

Hierzu wird insbesondere das Ergebnis der Auswertung der Meßwerte bzw. der Kurvenform einem Vergleich mit einem gespeicherten Vergleichswert oder einer Vergleichs-Kurvenform unterzogen, wobei das Vergleichsergebnis eine quantitative Bewertung des Vorkommens von Wasserinhaltsstoffen mit unterschiedlichem spezifischem Sauerstoffbedarf ermöglicht.

Für den realisierbaren Meßbereich und einen zeitsparenden Ablauf der Meßreihen ist wesentlich, daß die vorgegebenen Potential- oder Stromstärkewerte derart gewählt sind, daß die Wasserinhaltstoffe bei der elektrochemischen Oxidation nicht direkt oxidiert, sondern stattdessen hochreaktive Verbindungen gebildet werden, die ihrerseits die Wasserinhaltsstoffe unspezifisch oxidieren, insbesondere OH-Radikale und/oder Ozon. Bevorzugt liegen die eingestellten Potentialwerte im Bereich von 1600 bis 2300 mV (NHE) bzw. die Stromdichtewerte im Bereich von 0,1 bis 20 $\mu A/mm^2$.

Als Arbeitselektrolyt dient bevorzugt 0,1-molare Kalium- oder Natriumsulfat-oder Natriumperchloratlösung, als Material für die Gegenelektrode Platin oder Titan und als Referenzelektrode ein $Hg/HgSO_4$-System.

Das Meßverfahren ist ohne weiteres automatisierbar, indem die Einstellung einer Mehrzahl vorgegebener Potential-oder Stromstärkewerte und die Erfassung und Auswertung der jeweils zugehörigen Meßwerte nach einem gespeicherten Meßprogramm erfolgt.

Ein Verfahren zur Steuerung einer Wasserversorgungsanlage oder einer Abwasserentsorgungsanlage aufgrund des erfindungsgemäßen Meßverfahrens ist so ausgestaltet, daß anhand des Meß- bzw. Auswertungsergebnisses eine Stellgröße, insbesondere zur Steuerung von Schiebern zur Steuerung verschiedener Wasserströme, in der Anlage bestimmt wird. Zusätzlich oder alternativ kann die Zugabe bestimmter Hilfsstoffe und/oder die Durchführung von physikalischen Behandlungsschritten (Rühren. Sauerstoffzufuhr etc.) anhand der Meßergebnisse gesteuert werden.

Die Vorrichtung zur Durchführung des Verfahrens umfaßt in einer zweckmäßigen Ausführung eine Programmsteuereinrichtung zur automatischen Einstellung einer Mehrzahl vorgegebener Potential- oder Stromstärkewerte und Erfassung der zugehörigen Stromstärke- bzw. Potential-Meßwerte.

Weiterhin umfaßt sie in bevorzugter Ausbildung zur automatischen Meßwertverarbeitung eine Auswertungseinrichtung zum In-Beziehung-Setzen der Stromstärke- oder Potential-Meßwerte, die einen Auswertungs-Programmspeicher zur Speicherung mindestens eines Auswertungsalgorithmus und einen Datenspeicher zur Speicherung der Meßwerte sowie eine Verarbeitungseinheit aufweist, sowie eine Anzeigeeinheit zur Darstellung des Auswertungsergebnisses für den Bediener.

Für die Prozeßsteuerung bevorzugt ist weiterhin eine Ausführung, in der die Auswertungseinrichtung einen Vergleichswertpeicher und eine mit den Ausgängen der Verarbeitungseinheit und des Vergleichswertspeichers verbundene Vergleichereinheit aufweist, so daß qualitative und - in gewissem Umfang - auch belastungsartspezifisch quantitative Aussagen zur Belastungsstruktur anhand von Soll- bzw. Vergleichswerten gewonnen und speziell Veränderungen der Wasser- bzw. Abwasserzusammensetzung erfaßt werden können.

Eine vorteilhafte Anbindung an die Prozeßsteuerung einer Wasserversorgungs- oder Abwasserenstorgungsanlage wird dadurch gewährleistet, daß eine mindestens mittelbar mit dem Ausgang der Auswertungseinrichtung verbundene Steuerreinrichtung zur Ausgabe von Steuersignalen an Stellglieder, insbesondere Schieber- oder Dosiermittel-Stelleinrichtungen, in der Wasserversorgungs- oder Abwasserentsorgungsanlage vorgesehen ist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 eine schematische Darstellung einer Vorrichtung zur Durchführung eines Verfahrens gemäß einer Ausführungsform der Erfindung,

Figur 2 eine qualitative grafische Darstellung des Verlaufes von Meßkurven I = f(U) , die mit Ausführungsformen des erfindungsgemäßen Verfahrens aufgenommen werden können,

Figur 3 eine schematische Darstellung einer Vorrichtung nach einer weiteren Ausführungsform und

Figur 4 ein Funktions-Blockschaltbild einer Ausführrungsform der Auswertungseinrichtung 14b nach Fig. 1.

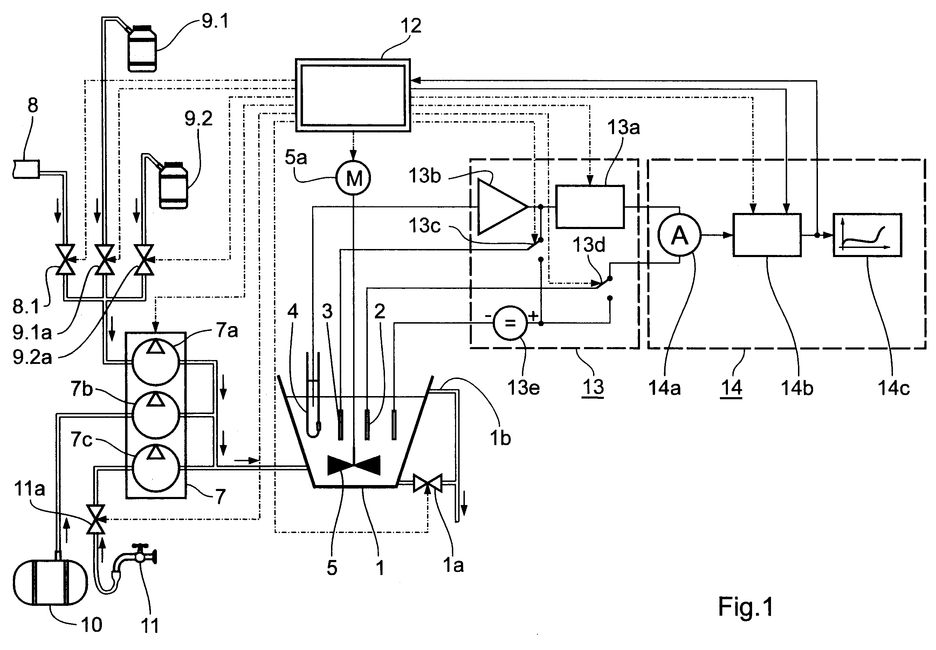

Bei der in der Figur 1 in Prinzipdarstellung dargestellten Meßanordnung befinden sich in einem Reaktionsgefäß 1, das von dem zu analysierenden Wasser bzw. Abwasser kontinuierlich durchströmt wird, drei Elektroden: eine Arbeitselektrode 2, eine Gegenelektrode 3 und eine Referenzelektrode 4. Das Reaktionsgefäß 1 ist mit einem Überlauf 1b, einem Ablaß-Magnetventil 1a und einem von einem Motor 5a betriebenen Rührer 5 ausgestattet.

Die Arbeitselektrode 2 weist einen metallischen Träger auf, auf den Bleidioxid abgeschieden ist. Die Gegenelektrode 3 besteht aus Platinblech; mit dem Effekt einer Kostenverringerung kann wahlweise auch Titanblech eingesetzt werden. Die Referenzelektrode 4 ist eine Bezugselektrode zweiter Art mit Diaphragma, die vorzugsweise als $Hg/HgSO_4$-System ausgeführt ist. Als Arbeitselektrolyt befindet sich 0,1-molare Kaliumsulfat- oder Natriumperchloratlösung im Reaktionsgefäß. Zur Regeneration der Eigenschaften der Elektroden 2 und 3 der Anordnung nach einer gewissen Reaktionsdauer ist eine Regenerationselektrode 6 vorgesehen.

Eine Dreikanalpumpe 7 dient zur Dosierung von Probenwasser aus einem Zulauf 8 bzw. von zwei verschiedenen Kalibrierlösungen mit bekanntem CSB aus entsprechenden Vorratsbehältern 9a, 9b (über den in der Figur oberen Pumpenkanal 7a) sowie zur Zugabe von Elektrolytkonzentrat aus einem Behälter 10 (über den mittleren Pumpenkanal 7b) und gegebenenfalls Verdünnungswasser aus einem Zulauf 11 (über den unteren Pumpenkanal 7c) für Proben mit sehr hohem CSB. Ein Magnetventi 8.1 leitet im Aktivierungsfall der Pumpe 7 Pro-benwasser zu, ein Magnetventil 9.1a leitet ihr wahlweise Kalibrierlösung für den unteren Kalibrierpunkt zu, ein Magnetventil 9.2a leitet ihr Kalibrierlösung für den oberen Kalibrierpunkt zu, und Verdünnungswasser wird ihr im Öffnungszustand eines Magnetventils 11a zugeleitet.

Zur automatischen Steuerung des Reaktions- und Meßprogramms ist ein Controller 12 vorgesehen, der in üblicher Weise (was in der Figur nicht gesondert gezeigt ist) einen Programm- und einen Datenspeicher aufweist. Durch das gespeicherte on-line anhand der Auswertungsergebnisse aktualisierte - Ablaufprogramm gesteuert, wird kontinuierlich Probenflüssigkeit vom Zulauf 8 in das Reaktionsgefäß 1 gefördert und mit Elektrolytkonzentrat aus dem Behälter 10 und gegebenenfalls Verdünnungswasser aus dem Zulauf 11 versetzt. Die Zugabe von konzentrierter Elektrolytlösung wird aufgrund der Probenwasser-Zulaufmenge gesteuert, in-dem die Förderleistung des oberen Pumpenkanals 7a und die des mittleren Pumpenkanals 7b geeignet aufeinander abgestimmt sind. Die Zugabe von Verdünnungswasser für Proben mit sehr hohem CSB wird anhand des Auswertungsergebnisses gesteuert, wobei der - sich aus der Aktivierungszeit des Magnetventils 11a und der Förderleistung des unteren Pumpenkanals 7c ergebende Verdünnungsgrad bei der Auswertung automatisch berücksichtigt wird.

Der Controller 12 nimmt aufgrund des Ablaufprogramms auch automatisch die Kalibrierung der Meßanordnung mit den zwei Kalibrierlösungen und die Regeneration der Arbeits- und der Gegenelektrode 2, 3 vor. Zur Kalibrierung wird das Reaktionsgefäß 1 über das Magnetventil 1a entleert, nacheinander aus den Gefäßen 9.1 bzw. 9.2 mit Kalibrierlösungen befüllt und jeweils eine Kalibrierung der - weiter unten genauer beschriebenen - Meßanordnung ausgeführt. Anschließend wird die automatische und kontinuierliche Messung fortgesetzt.

Die elektrische Schaltung zur Spannungsversorgung der Anordnung ist in einer separaten Einheit 13 vorgesehen, die eine steuerbare Spannungsquelle 13a umfaßt, die eine vorwählbare und auf den vorgewählten Wert stabilisierte Festspannung abgibt. Der steuerbaren Spannungsquelle 13a vorgeschaltet ist ein Impedanzwandler 13b mit extrem hochohmigem Eingang, der die Referenzelektrode 4 stromfrei hält. Ein Umschalter 13c dient zur Schaltung der Gegenelektrode 3 in den Regenerationsstromkreis und ein weiterer Umschalter 13d zur Umschaltung der Arbeitselektrode 2 zur Regenaration. Als Spannungsversorgung für den Regenerationsvorgang ist eine separate Konstantspannungsquelle 13e vorgesehen, mit der die Elektroden 2, 3 über die Umschalter 13c, 13d verbunden werden.

In den Stromkreis zwischen Arbeitselektrode 2 und Referenzelektrode 4 ist ein Mikroamperemeter 14a eingeschleift, das zusammen mit einer angeschlossenen Mikroprozessor-Auswertungseinheit 14b und einer Anzeigeeinheit 14c die eigentliche Meßanordnung 14 bildet. Die Auswertungseinheit 14b ist ein- wie ausgangsseitig mit dem Controller 12 verbunden, dem sie die aktuellen Meßergebnisse zur Aktualisierung der Programmablaufsteuerung übergibt und von dem sie zum einen Steuersignale zum Ablauf der Auswertung und zum anderen ein den aktuellen Verdünnungsgrad der Probenlösung reflektierendes Datensignal erhält.

Das Meßverfahren läuft wie folgt ab:

Nachdem das Reaktionsgefäß 1 befüllt ist und die Zuflußgrößen stabil sind, wird an der steuerbaren Spannungsquelle 13a über den Controller 12 ein erster vorgewählter Potentialwert eingestellt. Unter Einwirkung des speziellen Potentials werden an der Arbeitselektrode 2 die in der Wasserprobe gelösten Stoffe unspezifisch oxidiert, d.h. sie geben - nach Maßgabe ihrer Oxidierbarkeit beim eingestellten Potentialwert - Elektronen ab, was zu einem Stromfluß in der Elektrolytlösung führt. Die Oxidation erfolgt vorrangig indirekt, bei bestimmten Wasserinhaltsstoffen kann aber auch in nennenswertem Umfang eine direkte Oxidation auftreten. An der Gegenelektrode 3 findet ein entsprechender Reduktionsprozeß statt, wodurch Elektronen aufgenommen werden. Der durch die Lösung fließende Strom wird mittels des Mikroamperemeters 14a erfaßt und stellt ein Maß für die je Zeiteinheit bei dem vorgewählten Potential an den Elektroden umgesetzte Stoffmenge dar. Der erfaßte Stromwert wird in der Auswertungseinheit - die in einer Ausführungsform weiter unten genauer beschrieben wird - zunächst gespeichert und wahlweise auch auf der Anzeigeeinheit 14c angezeigt. Anschließend wird über den Controller ein zweiter Potentialwert eingestellt und die Messung bei dem neuen Wert wiederholt, wobei auch der hierbei erhaltene StromMeßwert gespeichert und angezeigt werden kann. Bei Vorliegen des zweiten Meßwertes ist aber auch bereits eine qualitative Auswertung in einer ersten Näherung möglich.

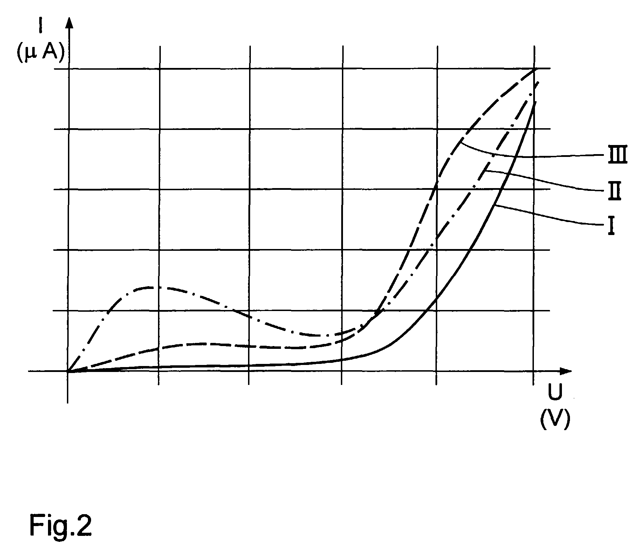

Deren Prinzip soll nachfolgend anhand der Fig. 2 erläutert werden, die eine qualitative grafische Darstellung des Verlaufes dreier Strom-Spannungs-Kurven I, II und III zeigt, die mit dem oben beschriebenen Verfahren an Abwässern mit unterschiedlich oxidierbaren Inhaltsstoffen unter schrittweiser Veränderung des Potentials U aufgenommen werden können. Kurve I symbolisiert die Meßergebnisse für ein Abwasser mir insgesamt geringer Oxidierbarkeit, d.h. niedrigem CSB. Kurve II repräsentiert ein Abwasser mit einem hohen Gehalt an leicht oxidierbaren Inhaltsstoffen, während Kurve III für ein Abwasser mit einem hohem Gehalt an schwer oxidierbaren Inhaltsstoffen steht.

Aus diesen Beispielen wird erkennbar, daß insbesondere die Aufnahme regelrechter Meßkurven, aber in erster Näherung auch bereits die Erfassung der Stromstärke bei einigen wenigen ausgewählten Potentialwerten, eine wesentlich differenziertere Aussage über die Oxidierbarkeit und damit den chemischen Sauerstoffbedarf von Wasser oder Abwasser erlaubt als eine Messung bei nur einem Potentialwert. Unter gewissen Voraussetzungen ist auf dieser Basis grundsätzlich sogar eine kontinuierliche Bestimmung des Gehaltes an bestimmten Inhaltsstoffen möglich.

Aus den dargestellten Kurven ist auch ersichtlich, daß anstelle der bekannten potentiostatischen Messung (Konstanthaltung von U bei Aufnahme eines Meßpunktes) auch ein als "galvanostatische Messung" zu bezeichnendes Verfahren anwendbar ist, bei dem - für einen Meßpunkt - der Stromfluß durch die Lösung konstant gehalten und der sich einstellende Potentialwert ermittelt wird. Durch sukzessive Einstellung verschiedener Stromwerte ist damit in analoger Weise wie oben beschrieben ein Abtasten des die Oxidierbarkeit der Probe reflektierenden Kurvenverlaufes möglich.

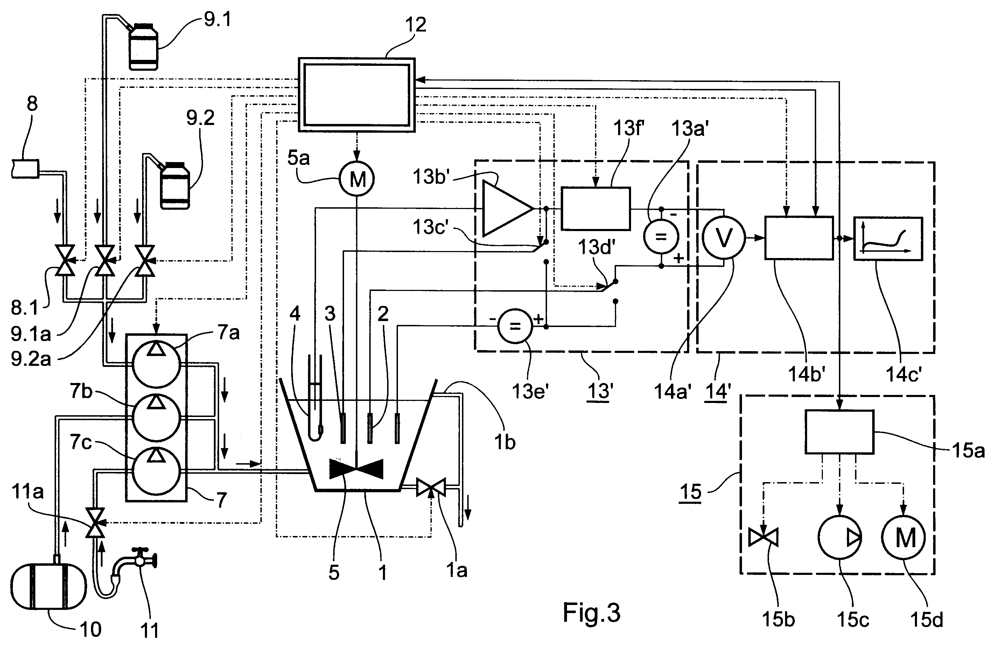

Eine entsprechend modifizierte Reaktions- und Meßanordnung zeigt Fig. 3. Mit Ausnahme der nachfolgend erwähnten Teile der elektrischen Schaltung und der eigentlichen Meßanordnung - die daher insgesamt eingestrichene Bezugsziffern erhielten - stimmen die Komponenten mit den in Fig. 1 gezeigten und oben beschriebenen überein. An die Stelle der gesteuerten Spannungsquelle tritt eine Spannungsquelle 13a' mit einer Stromstabilisatorschaltung 13e', die gemeinsam eine (steuerbare) Konstantstromquelle bilden. Die sich im Ergebnis des Regelvorganges über der Spannungsquelle 13a' einstellende Effektivspannung wird durch ein Voltmeter 14a' erfaßt. Dies sukzessive Einstellung unterschiedlicher Werte (hier: I-Werte) und die Handhabung der entsprechenden Meßwerte (hier: U-Werte) entspricht grundsätzlich dem Vorgehen bei der potentiostatischen Messung.

In Fig. 3 ist auch symbolisch dargestellt, daß das Auswertungsergebnis, d.h. das Ausgangssignal der Auswertungseinheit 14b', zur Ableitung der Steuergröße für eine Steuerung 15 einer Abwasseraufbereitungsanlage dienen kann. Das Ausgangssignal der Auswertungseinheit 14b' wird einer Prozeßsteuereinheit 15a zugeführt, die es gemäß einem gespeicherten Steuerprogramm in Steuersignale zur Betätigung von Schiebern bzw. Ventilen für verschiedene Abwasserströme - in der Figur symbolisiert durch das Absperrventil 15b - , Dosiereinrichtungen für Zuschlagstoffe - symbolisiert durch die Pumpe 15c - und/oder Antriebe für mechanische Aufbereitungsmittel - symbolisiert durch den Motor 15c - umsetzt. Auf diese Weise kann in Abhängigkeit vom erfindungsgemäß gewonnenen Auswertungsergebnis die Güte von Abwasser in einem Entsorgungsweg durch Steuerung der Mischung und/oder Einleitung in Abhängigkeit von der Wassergüte und/oder Steuerung der physikalischen und chemischen Vorbehandlung der Mischungskomponenten automatisch gesteuert werden.

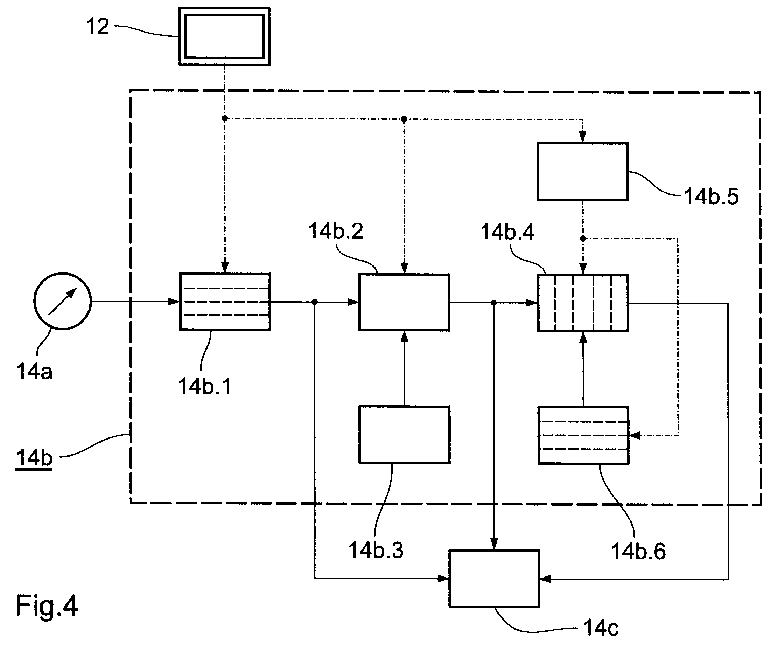

Figur 4 ist ein Funktions-Blockschaltbild einer Ausführungsform der Auswertungseinrichtung 14b aus Fig. 1 (der die Einheit 14b' aus Fig. 3 - mit dem Unterschied, daß dort Spannungs-Meßwerte ausgewertet werden - vollständig entsprechen kann).

Diese umfaßt einen über einen Dateneingang mit dem Meßgerät - hier dem Mikroamperemeter 14a - und über einen Steuereingang mit dem Controller 12 verbundenen Meßwertspeicher 14b.1, in dem die sukzessive aufgenommenen (Strom-)Meßwerte unter der Adresse des jeweiligen (Spannungs-)Einstellwertes abgelegt werden. Eine Meßdaten-Verarbeitungseinheit 14b.2 verarbeitet - sobald mehrere Meßwerte vorliegen - unter Ablaufsteuerung durch den Controller 12 die gespeicherten Meßwerte mittels eines in einem Auswertungsprogrammspeicher 14b.3 gespeicherten Algorithmus. Parallel werden die zur Verarbeitung abgerufenen gespeicherten Meßwerte der Anzeigeeinheit 14c zugeführt, wo ihre Darstellung beispielsweise einen Kurvenverlauf in Art der in Fig. 2 schematisch dargestellten Kurven ergibt.

Das Ausgangssignal der Verarbeitungseinheit 14b.2 stellt - je nach Umfang der Meßreihe und Auswertungsalgorithmus - bereits eine qualitative Aussage über das Vorliegen leicht und/oder schwer oxidierbarer Inhaltsstoffe dar und wird ebenfalls der Anzeigeeinheit 14c zugeführt. Weiterhin gelangt es zu einem Eingang einer mehrstufigen Vergleichereinheit 14b.4, deren anderer Eingang mit einem Mehrbereichs-Vergleichswertspeicher 14b.6 verbunden ist. Gesteuert durch eine separate Ablaufsteuerung 14b.5, wird das primäre Auswertungsergebnis einem mehrstufigen Vergleich mit Soll- oder Vergangenheitswerten als Sekundärauswertung unterzogen. In deren Ergebnis können insbesondere auch quantitative Aussagen hinsichtlich des Gehaltes an bestimmten Wasserinhaltsstoffen und gegebenenfalls. dessen zeitlicher Entwicklung bereitgestellt werden. Auch diese Ergebnisse werden angezeigt und können überdies - im Sinne einer Anordnung nach Fig. 3 - unmittelbar der Prozeßsteuerung einer Wasserversorgung- oder Abwasseraufbereitungsanlage zugeführt werden.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

**Patentansprüche**

1. Verfahren zur Bestimmung des chemischen Sauerstoffbedarfs von Wasser bzw. Abwasser über die elektrochemische Oxidation von deren Inhaltsstoffen an einer Bleidioxid-Arbeitselektrode, **dadurch gekennzeichnet**, daß

   - die Bleidioxid-Arbeitselektrode zeitlich aufeinanderfolgend bei mindestens zwei verschiedenen vorgegebenen Potentialen betrieben wird,
   - der bei jedem vorgegebenen Potential durch das Wasser bzw. Abwasser fließende Strom gemessen wird und
   - die Strom-Meßwerte in eine vorgegebene Beziehung zueinander gesetzt werden, insbesondere ihr Quotient oder ihre Differenz oder eine Meßkurve $I = f(U)$ gebildet wird, wobei das Ergebnis des In-Beziehung-Setzens eine Aussage über das Vorkommen von Wasserinhaltsstoffen mit unterschiedlichem spezifischem Sauerstoffbedarf darstellt.

2. Verfahren zur Bestimmung des chemischen Sauerstoffbedarfs von Wasser bzw. Abwasser über die elektrochemische Oxidation von deren Inhaltsstoffen an einer Bleidioxid-Arbeitselektrode, **dadurch gekennzeichnet**, daß

   - zeitlich aufeinanderfolgend mindestens zwei verschiedene vorgegebene Stromstärkewerte eines durch das Wasser bzw. Abwasser fließenden Stromes eingestellt werden,
   - das bei jeder vorgegebenen Stromstärke sich an der Bleidioxid-Arbeitselektrode einstellende Potential gemessen wird und
   - die Potential-Meßwerte in eine vorgegebene Beziehung zueinander gesetzt werden, insbesondere ihr Quotient oder ihre Differenz oder eine Meßkurve $U = f(I)$ gebildet wird, wobei das Ergebnis des In-Beziehung-Setzens eine Aussage über das Vorkommen von Wasserinhaltsstoffen mit unterschiedlichem spezifischem Sauerstoffbedarf darstellt.

3. Verfahren zur Bestimmung des chemischen Sauerstoffbedarfs von Wasser bzw. Abwasser über die elektrochemische Oxidation von deren Inhaltsstoffen an einer Bleidioxid-Arbeitselektrode, **dadurch gekennzeichnet**, daß

   - ein vorgegebener Stromstärkewert eines durch das Wasser bzw. Abwasser fließenden Stromes eingestellt und
   - das bei der vorgegebenen Stromstärke sich an der Bleidioxid-Arbeitselektrode einstellende Potential gemessen wird, wobei der Potential-Meßwert eine Aussage über das Vorkommen von Wasserinhaltsstoffen mit unterschiedlichem spezifischem Sauerstoffbedarf darstellt.

4. Verfahren nach einem der vorangehenden Ansprüche, **dadurch gekennzeichnet**, daß ein im Ergebnis des In-Beziehung-Setzens erhaltener Ist-Wert oder eine Ist-Kurvenform oder der Potential-Meßwert einem Vergleich mit einem gespeicherten Vergleichswert oder einer Vergleichs-Kurvenform unterzogen wird, wobei das Vergleichsergebnis eine quantitative Bewertung des Vorkommens von Wasserinhaltsstoffen mit unterschiedlichem spezifischem Sauerstoffbedarf ermöglicht.

5. Verfahren nach einem der Ansprüche 1, 2 oder 4, **dadurch gekennzeichnet**, daß die Einstellung einer Mehrzahl vorgegebener Potential- oder Stromstärkewerte und die Erfassung und Auswertung der jeweils zugehörigen Meßwerte nach einem gespeicherten Meßprogramm erfolgt.

6. Verfahren nach einem der vorangehenden Ansprüche, **dadurch gekennzeichnet**, daß die vorgegebenen Potential- oder Stromstärkewerte derart gewählt sind, daß die Wasserinhaltstoffe bei der elektrochemischen Oxidation nicht direkt oxidiert, sondern stattdessen hochreaktive Verbindungen gebildet werden, die ihrerseits die Wasserinhaltsstoffe unspezifisch oxidieren, insbesondere OH-Radikale und/oder Ozon.

7. Verfahren nach Anspruch 6, **dadurch gekennzeichnet**, daß Potentialwerte im Bereich von 1600 bis 2300 mV bzw. Stromdichtewerte im Bereich von 0,1 bis 20 $\mu$A/mm$^2$ eingestellt werden.

8. Verfahren nach einem der vorangehenden Ansprüche, **dadurch gekennzeichnet**, daß als Arbeitselektrolyt 0,1-molare Kalium- oder Natriumsulfat- oder Natriumperchloratlösung, als Material für die Gegenelektrode Platin oder Titan und als Referenz- elektrode ein Hg/HgSO$_4$-System eingesetzt wird.

9. Verfahren zur Steuerung einer Wasserversor- gungsanlage oder einer Abwasserentsorgungsan- lage unter Einschluß eines Verfahrens nach einem der vorangehenden Ansprüche, **dadurch gekennzeichnet**, daß anhand des Ergebnisses des In-Beziehung- Setzens oder des Potential-Meßwertes eine Stellgröße, insbesondere zur Steuerung von Schiebern, Dosiermitteln oder Antriebsmotoren, in der Wasserversorgungs- oder Abwasserentsorgungsanlage bestimmt wird.

10. Vorrichtung zur Durchführung des Verfahrens nach Anspruch 1 oder einem der Ansprüche 4 bis 9, mit einer Spannungsquelle, einem Wasser- bzw. Abwasserbehälter, einer Bleidioxid-Arbeitselek- trode, einer stromfrei gehaltenen Referenzelek- trode, einer zur Referenzelektrode über einen Widerstand parallelgeschalteten Gegenelektrode und einem in Serienschaltung zwischen der Gegen- und der Arbeitselektrode angeordneten Strommeßgerät, **gekennzeichnet durch** Mittel zur Einstellung unterschiedlicher vorgegebener Potentialwerte an der Spannungsquelle.

11. Vorrichtung zur Durchführung des Verfahrens nach Anspruch 2 oder einem der Ansprüche 4 bis 9, mit einer Spannungsquelle, einem Wasser- bzw. Abwasserbehälter, einer Bleidioxid-Arbeitselek- trode, einer stromfrei gehaltenen Referenzelek- trode, einer zur Referenzelektrode über einen Widerstand parallelgeschalteten Gegenelektrode und einem einem parallel zur Spannungsquelle geschalteten Spannungsmeßgerät, **gekennzeichnet durch** Mittel zur Einstellung unterschiedlicher vorgegebener Stromstärkewerte zwischen Arbeits- und Gegenelektrode.

12. Vorrichtung nach Anspruch 10 oder 11, **gekennzeichnet durch** eine Programmsteuereinrichtung zur automatischen Einstellung einer Mehrzahl vorgegebener Potential- oder Stromstärkewerte und Erfassung der zugehörigen Stromstärke- bzw. Potential-Meßwerte.

13. Vorrichtung nach einem der Ansprüche 10 bis 12, **gekennzeichnet durch** eine Auswertungseinrichtung zum InBeziehung-Setzen der Stromstärke- oder Potential-Meßwerte, die einen Auswertungs- Programmspeicher zur Speicherung mindestens einer vorgegebenen Beziehung und einen Datenspeicher zur Speicherung der Meßwerte sowie eine Verarbeitungseinheit aufweist, sowie eine Anzeige- einheit zur Darstellung des Auswertungsergebnis- ses.

14. Vorrichtung nach Anspruch 13, **dadurch gekennzeichnet**, daß die Auswertungseinrichtung einen Vergleichswertpeicher und eine mit den Ausgängen der Verarbeitunsgeinhiet und des Vergleichswertspeichers verbundene Vergleichereinheit aufweist.

15. Vorrichtung nach Anspruch 13 oder 14 zur Durchführung der Verfahrens nach Anspruch 9, **dadurch gekennzeichnet**, daß eine mindestens mittelbar mit dem Ausgang der Auswertungseinrichtung verbundene Steuereinrichtung zur Ausgabe von Steuersignalen an Stellglieder, insbesondere Schieber- Stelleinrichtungen, in der Wasserversorgungs- oder Abwasserentsorgungsnlage vorgesehen ist- vorgesehen ist.

Fig.1

EP 0 834 739 A2

8

Fig.2

Fig.3

Fig.4

EP 0 834 739 A2